# EUROPEAN PATENT APPLICATION

(11) **EP 2 666 924 A2**
(43) Date of publication of application: **27.11.2013**
(21) Application number: 11856301.4
(22) Date of filing: 27.12.2011
(51) Int. Cl.: E04C 3/02, E04B 1/08

(54) **TRUSS STRUCTURE USING A MATERIAL HAVING A PI-SHAPED CROSS-SECTION AS AN UPPER CHORD**

(30) Priority: 17.01.2011 KR 20110004554
(71) Applicant: Chang, Kwang Yoon, Seoul 138-050 (KR)
(72) Inventor: Chang, Kwang Yoon, Seoul 138-050 (KR)
(74) Representative: Caspary, Karsten
(86) International application number: PCT/KR2011/010162
(87) International publication number: WO 2012/099346

(57) **Abstract**

The present invention relates to a pi truss structure. A pi truss structure, in which a bracing part is coupled to a coupling groove defined on the inside of each upper and lower pi parts, respectively, and wherein web members having two rows of the pi parts are disposed and coupled so as to face each other at a right angle within a plate-shaped flange member, and the coupling groove having a 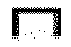 shape is defined inward in the lengthwise direction. Also, one side of each of the web members is disposed on the flange member, the other side being bent outward so as to form a reinforcing flange part. The coupling grooves of the upper and lower pi parts are disposed facing each other so as to couple coupling members to the coupling grooves, respectively. Also, the bracing part is coupled to the coupling members.

## Description

### [Technical Field]

The present invention relates to a truss structure using a pi-shaped cross section as chord members and, more particularly, to a truss structure in which upper and lower portions of a bracing part are coupled to pi-shaped coupling grooves formed inwardly by a flange member and web members in two rows coupled to the inside of the flange member.

### [Background Art]

In general, a truss means a frame structure, in which several straight members are connected at joints to be arranged in the form of one or more triangles, and is used as a structural member in a roof framing or floor framing of a structure.

The truss comprises an upper chord, a lower chord, a vertical member, and a diagonal member and is commonly used in a building or civil engineering structure. When an axial stress occurs in the upper chord and the lower chord, if the center-to-center distance between the upper chord and the lower chord is greater, the axial stress is reduced, while the rigidity of the truss increases.

The resisting force of the upper chord or lower chord in the truss, which is subjected to a compressive force, is determined by the slenderness ratio, and thus when the slenderness ratio of a main shaft by the length of the vertical member or the diagonal member is closer to the slenderness ratio of a countershaft by the buckling length of a compression member that compresses a member installed in an orthogonal direction in the truss, the efficiency of the member increases.

For this reason, in a conventional truss, it is most economical to use two angles as the upper chords or lower chords, but the standard of the angles is limited. Thus, the angles are not suitable for a high load or long span truss and it is difficult to apply anticorrosive and fire retarding paints, which is problematic.

In the case where a single T-section steel is used as the upper chord and the lower chord of the truss, the width-thickness ratio of a ready-made stem (web) exceeds the limit, and the section efficiency is reduced. Therefore, there is a method forming a truss having a T-shaped cross section by welding steel plates, but a separate member should be installed to control out-of-plane buckling, and thus the truss is not suitable for the high load truss.

When the truss is made of a circular steel pipe, the truss is relatively lightweight, but the radius of gyration of two axes is the same, and thus the distance between subtrusses determines the resisting force of the member, which decreases the efficiency and reduces the bending strength. As a result, such a truss is not suitable for the high load or long span truss.

Accordingly, in the case of the long span truss, a triangular steel pipe truss, in which the upper chord or lower chord which is subjected to compression is made of two steel pipes, is widely used. However, in this steel pipe truss structure, the vertical members and diagonal members as branch pipes are welded to the upper chord or lower chord as a main pipe, and thus a high local stress is generated in the main pipe. Thus, if the axial force of the vertical members or diagonal members is high, the application is limited, and the vertical members and diagonal members should be welded in a factory and then transported. As a result, the production cost increases and there are difficulties in the transportation.

There is an under-tension structure in which the bottom of a steel pipe trust is tensioned so as to be applied to the long span truss, but the cost for the tension increases.

In a conventional high load truss, a H-section steel is generally used as the upper chord or lower chord, but in the case of the long span truss, the stress due to the truss weight is high, which reduces the efficiency, which is problematic.

### [Disclosure]

### [Technical Problem]

Accordingly, the present invention has been made to solve the above-described problems, and an object of the present invention is to provide a truss structure in which upper and lower pi parts, i.e., upper and lower chords, installed at the top and bottom of a truss have a pi-shaped cross section such that the center of the cross section is closer toward a flange to increase the efficiency of the cross section, thus increasing the rigidity. In particular, the bending strength is increased such that a high load truss can be supported and, at the same time, a long span truss can also be supported.

### [Technical Solution]

To accomplish the above objects of the present invention, an aspect of the present invention provides a pi truss structure, in which a bracing part is coupled to a coupling groove formed on the inside of each of pi-shaped upper and lower pi parts, wherein each of the pi parts is configured in a manner that web members in two rows are provided to face each other at right angles and coupled to the inside of a plate-shaped flange member such that a 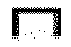 -shaped coupling groove is formed on the inside of the pi part in a longitudinal direction, wherein one side of each of the web members is formed on the flange member and the other side thereof is bent outward to form a reinforcing flange part, wherein the coupling grooves of the upper and lower pi parts are provided to face each other such that coupling members are coupled to the coupling grooves, respectively, and the bracing part is coupled to the coupling members.

Moreover, one side of each of the web members facing the flange member is bent in the same shape as the reinforcing flange part to form an upper reinforcing flange part.

Furthermore, one side of one of the web members at left and right sides facing the flange member is connected to one side of the other web member by a horizontal reinforcing member.

In addition, the coupling member comprises a first coupling piece and a second coupling piece, which are coupled by welding, and the first coupling piece and the second coupling piece comprise coupling holes, respectively.

Additionally, reinforcing pieces are inserted into the inside of the coupling groove in a manner to face each other and coupled thereto by welding, and each of the reinforcing pieces has a shape selected from the group consisting of "-", " 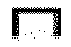 ", "┌" and "┐".

### [Advantageous Effects]

According to the present invention, the cross efficiency of the upper and lower pi parts, i.e., the upper and lower chords, installed at the top and bottom of a truss is increased such that the rigidity and bending strength can be increased, thus stably supporting a high load truss as well as a long span truss.

Moreover, the radius of gyration of countershafts of pi-shaped pi parts is increased to reduce the effect of lateral bucking and reduce the amount of members.

As a result, the production, transportation, and installation of the truss can be facilitated, thus reducing the working time and costs.

### [Description of Drawings]

FIG. 1 is a perspective view showing a pi truss structure according to the present invention.
FIG. 2 is an enlarged perspective view showing a major portion of the pi truss structure according to the present invention.
FIG. 3 is an exploded perspective view showing the disassembled pi truss structure according to the present invention.
FIG. 4 is a cutaway side view showing the major portion in an assembled state of the pi truss structure according to the present invention.
FIG. 5 is a view showing an example of the use of the pi truss structure according to the present invention.
FIGS. 6A to 6C are front views showing pi truss structures in accordance with other embodiments of the present invention.

### Description of Reference Numerals

10: pi part 11: flange member
12: web member 13: coupling groove
14: reinforcing flange part
20: bracing part 30: coupling member

### [Mode for Invention]

Hereinafter, preferred embodiments of the present invention will be described in detail with reference to the accompanying drawings.

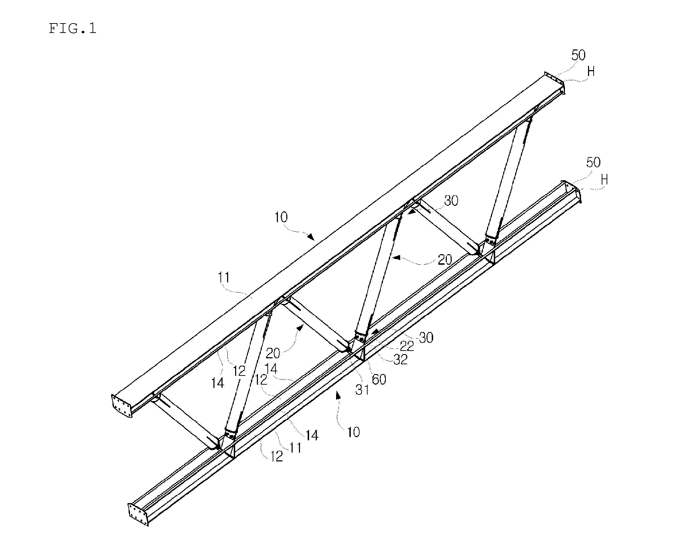

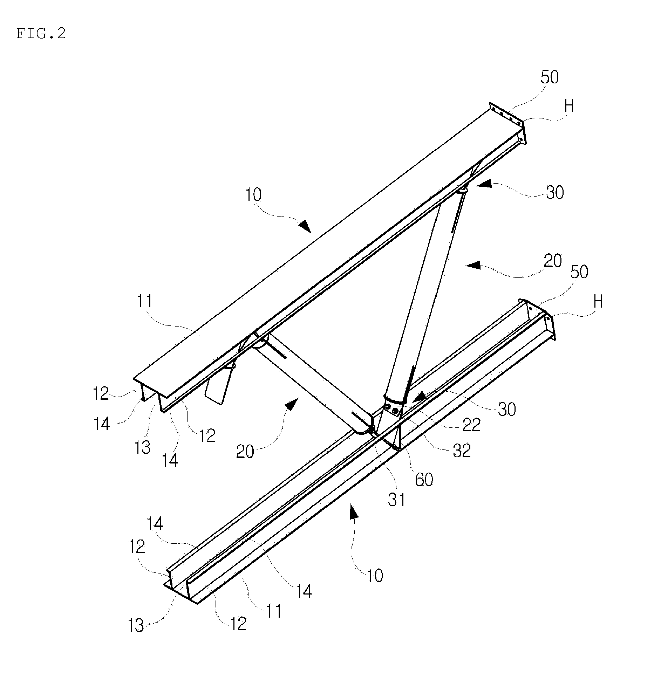

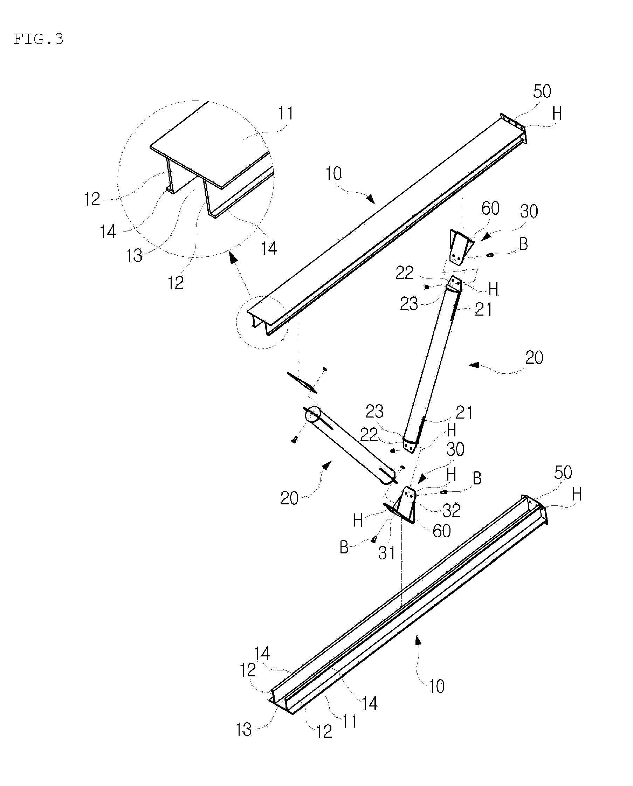

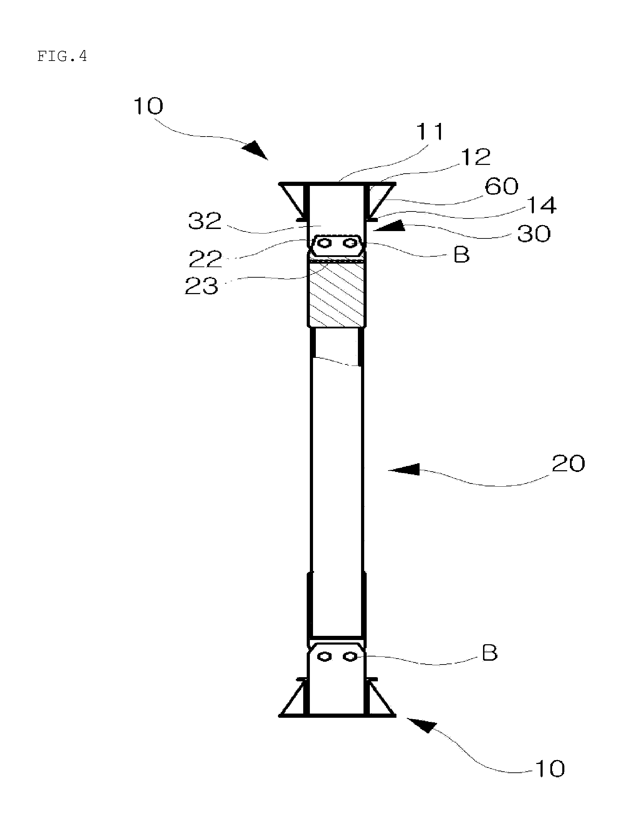

As shown in FIGS. 1 to 5, a pi truss structure according to the present invention is configured in a manner that a bracing part 20 is coupled to a coupling groove 13, which is formed on the inside of each pi-shaped upper and lower pi parts 10 by a coupling member 30.

Here, the upper and lower pi parts 10 are configured in a manner that web members 12 in two rows, which are spaced at regular intervals, are coupled to the inside of a plate-shaped flange member 11 in a manner to face each other at right angles such that a 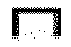 -shaped coupling groove 13 is formed between them in a longitudinal direction.

One side of each of the web members 12 in two rows is in direct contact with the flange member 11 and coupled thereto by welding, and the other side of each of the welded web members 12 is bent outward to form a reinforcing flange part 14.

As the center of the pi-shaped cross section is closer toward the flange member 11, the center-to-center distance between the cross sections located at the top and bottom is maximized at the same height, and thus the stress is minimized, the strength is maximized, and the bent portion forms a flange, thus significantly increasing the bending strength. Here, in the case where a steel plate, which forms the flange member 11, the web members 12, and the reinforcing flange parts 14, are configured such that the width varies depending on the length, it is possible to provide a more economical design of a truss structure in which the stress varies depending on its length.

In a state where the coupling grooves 13 formed on the upper and lower pi parts 10 are arranged to face each other, the coupling members 30 are respectively installed in the coupling grooves 13 formed on the inside of the upper and lower pi parts 10, and the upper and lower portions of each of the bracing parts 20 are coupled to the coupling members 30 located at the top and bottom.

End plates 50 each having coupling holes H are coupled to both ends of each of the upper and lower pi parts 10 in a manner to face each other such that the upper and lower pi parts 10 can be continuously installed in the longitudinal direction.

The bracing parts 20 are respectively coupled to the upper and lower pi parts 10 by means of the coupling members 30. A slit groove 21 is formed in the axial direction in the middle of both ends, respectively, and a third coupling piece 22 having coupling holes H is inserted into the slit groove 21 and coupled thereto by welding.

Semicircular pieces 23 are welded to both sides of each of the third coupling pieces 22, which are inserted into both ends of the bracing part 20 and coupled thereto by welding, in a manner to face each other and coupled thereto by welding, thus increasing the strength of the coupling and, at the same time, preventing rainwater or foreign materials from entering from the outside.

Each of the coupling members 30 allows the coupling groove 13 and the bracing part 20 to be connected by means of bolts B. A second coupling piece 32 is coupled to a first coupling piece 31 by welding, and the welded first coupling piece 31 and second coupling piece 32 are inserted and coupled to the inside of the coupling groove 13 by welding.

Coupling holes H are formed in each of the first coupling piece 31 and the second coupling piece 32 such that the third coupling pieces 22, provided on the upper and lower portions of each of the bracing parts 20 and being in contact therewith are coupled thereto by means of bolts B.

Two bracing parts 20 are coupled to the coupling members 30 provided on the upper pi part 10 and the coupling members 30 provided on the lower pi part 10.

Triangular reinforcing pieces 60 are coupled to both sides of each of the web members 12, which are respectively formed on the upper and lower pi parts 10, by welding.

Reinforcing pieces 40 each having a predetermined length are inserted into the inside of the coupling grooves 13, formed on the upper and lower pi parts 10, in a manner to face each other and coupled thereto by welding, thus partially controlling the strength.

Each of the reinforcing pieces 40 has a shape selected from the group consisting of "-", " 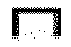 " , "┌" and "┐" such that the reinforcing pieces 40 can easily face each other in the coupling grooves 13 formed on the upper and lower pi parts 10. That is, in the regions where the bracing parts 20 are coupled to the upper and lower pi parts 10, the stress gradually increases or decreases depending on the length of the regions. Therefore, a region having a high stress is reinforced by the reinforcing pieces 40, and the other regions can bear the stress with non-reinforced cross sections.

In the above-described pi truss, first of all, the web members 12 having the reinforcing flange parts 14 bent outward are arranged in two rows to face each other at right angles and coupled to the inside of the plate-shaped flange member 11, made of a steel plate, by welding. These web members 12 may be made of angles or formed by bending steel plates.

Then, the coupling member 30, to which the first coupling piece 31 and the second coupling piece 32 are coupled by welding, is inserted into the inside of the coupling groove 13 formed in the longitudinal direction and coupled thereto by welding. Here, the triangular reinforcing pieces 60 are coupled to both sides of each of the web members 12, which are respectively formed on the upper and lower pi parts 10, by welding.

Next, the reinforcing pieces 40 each having a length between the bracing parts 20 are inserted into the inside of the coupling grooves 13, formed on the upper and lower pi parts 10, in a manner to face each other and coupled thereto by welding, and then the end plates 50 each having the coupling holes H are coupled to both ends of each of the upper and lower pi parts 10 in a manner to face each other by welding.

Next, the coupling grooves 13, formed on the upper and lower pi parts 10, are arranged to face each other, and then each of the third coupling pieces 22, provided on the upper and lower portions of the bracing part 20 and facing the first coupling piece 31 and the second coupling piece 32, is coupled through the coupling holes H by means of bolts B.

Next, the process of coupling each of the third coupling pieces 22, provided on the upper and lower portions of the bracing part 20 and facing the first coupling piece 31 and the second coupling piece 32, through the coupling holes H by means of bolts B is repeated such that the bracing parts 20 are coupled to all of the first coupling pieces 31 and the second coupling pieces 32 provided on the upper and lower pi parts 10.

Next, the process of arranging the end plates 50 having the coupling holes H face each other and coupling the end plates 50 to both ends of the upper and lower pi parts 10 by bolting in a state where the bracing parts 20 are coupled to all of the first coupling pieces 31 and the second coupling pieces 32 provided on the upper and lower pi parts 10 is repeated, thus constructing a long span truss.

Meanwhile, in the present invention, all of the first coupling pieces 31 and the second coupling pieces 32, provided on the upper and lower pi parts 10, are open to have the same angle in both directions such that the bracing parts 20 are coupled in a zigzag manner, but the first coupling pieces 31 or the second coupling piece 32 may be arranged vertically such that one of two bracing parts 20 coupled to the coupling members 30 can be installed vertically.

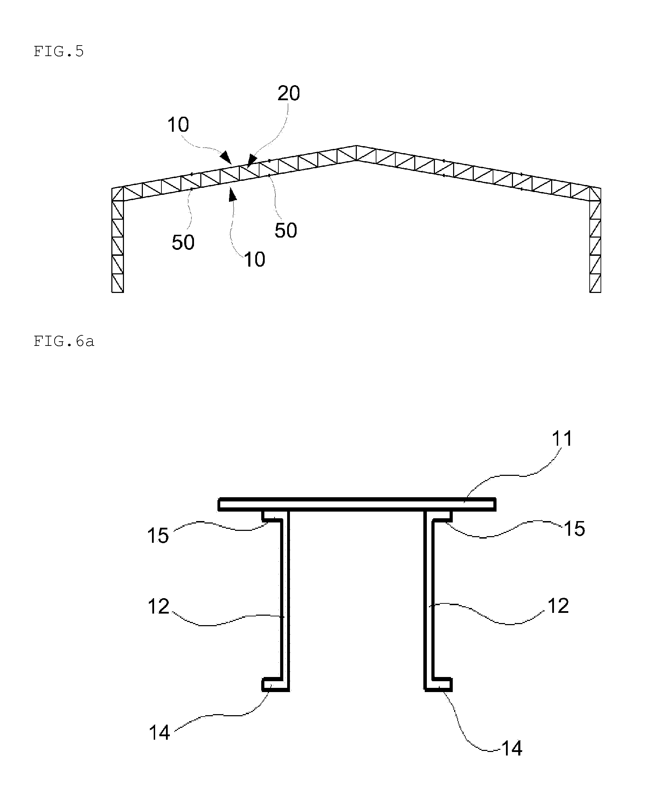

Moreover, in the present invention, one side of each of the web members 12 coupled to the inside of the flange member 11 is brought into direct contact with the flange member 11 at a right angle and coupled thereto by welding, but the one side of the web member 12 facing the flange member 11 may be bent in the same shape as the reinforcing flange part 14, thus forming an upper reinforcing flange part 15 as shown in FIG. 6A. In this case, the strength of the portion to which the flange member 11 is coupled by welding can be further increased.

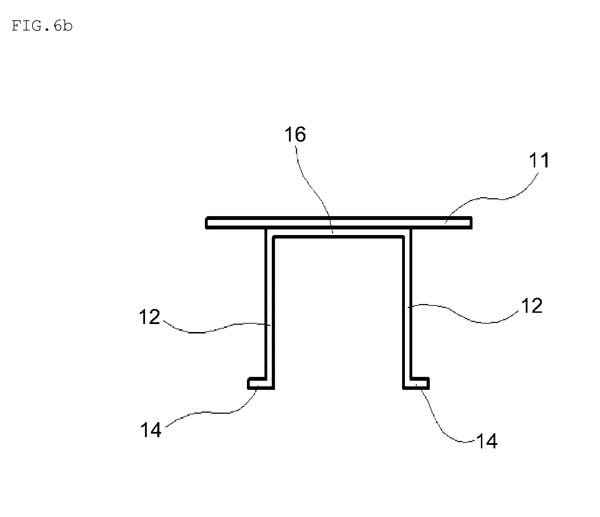

Meanwhile, in the present invention, one side of each of the web members 12 coupled to the inside of the flange member 11 is brought into direct contact with the flange member 11 at a right angle and coupled thereto by welding, but one side of one of the web members 12 at left and right sides facing the flange member 11 may be connected to one side of the other web member 12 by a horizontal reinforcing member 16 as shown in FIG. 6B.

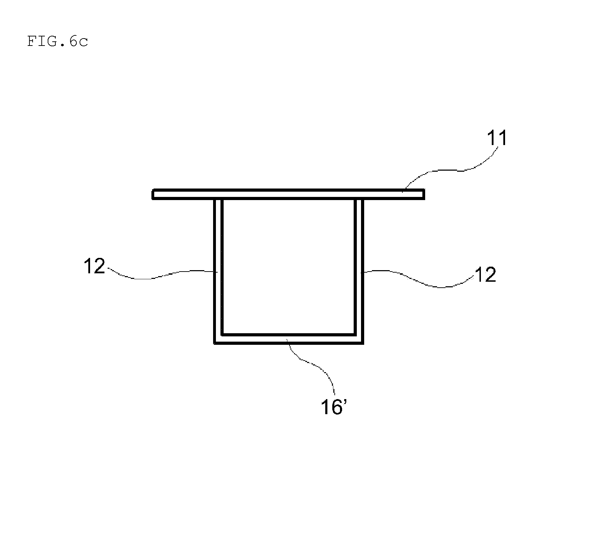

Furthermore, in the present invention, one side of each of the web members 12 coupled to the inside of the flange member 11 is brought in to direct contact with the flange member 11 at a right angle and coupled thereto by welding, but the other side of each of the web members 12 may be connected by another horizontal reinforcing member 16 as shown in FIG. 6C. In this case, a box-shaped space is created at the bottom of the flange member 11, thus obtaining more structurally stable strength.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

## Claims

1. A pi truss structure, in which a bracing part is coupled to a coupling groove formed on the inside of each of pi-shaped upper and lower pi parts, wherein each of the pi parts is configured in a manner that web members in two rows are provided to face each other at right angles and coupled to the inside of a plate-shaped flange member such that a 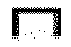 -shaped coupling groove is formed on the inside of the pi part in a longitudinal direction, wherein one side of each of the web members is formed on the flange member and the other side thereof is bent outward to form a reinforcing flange part, wherein the coupling grooves of the upper and lower pi parts are provided to face each other such that coupling members are coupled to the coupling grooves, respectively, and the bracing part is coupled to the coupling members.

2. The pi truss structure of claim 1, wherein one side of each of the web members facing the flange member is bent in the same shape as the reinforcing flange part to form an upper reinforcing flange part.

3. The pi truss structure of claim 1, wherein one side of one of the web members at left and right sides facing the flange member is connected to one side of the other web member by a horizontal reinforcing member.

4. The pi truss structure of claim 1, wherein the coupling member comprises a first coupling piece and a second coupling piece, which are coupled by welding, and the first coupling piece and the second coupling piece comprise coupling holes, respectively.

5. The pi truss structure of claim 1, wherein reinforcing pieces are inserted into the inside of the coupling groove in a manner to face each other and coupled thereto by welding, and each of the reinforcing pieces has a shape selected from the group consisting of "-", " 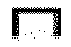 ", "┌" and "┐".
